# EUROPEAN PATENT APPLICATION

(11) **EP 2 444 394 A1**
(43) Date of publication of application: **25.04.2012**
(21) Application number: 11184304.1
(22) Date of filing: 07.10.2011
(51) Int. Cl.: C07D 211/90

(54) **Process for the preparation of amorphous form of lercanidipine HCI**

(30) Priority: 21.10.2010 IN MU29132010
(71) Applicant: Alembic Pharmaceuticals Limited, Vadodara 390003 GUJ (IN)
(72) Inventor: Raman, Jayaraman Venkat, 390003 Vadodara (IN); Tomer, Sanjiv, 390003 Vadodara (IN); Rana, Piyush, 390003 Vadodara (IN); Dolia, Mitul, 390003 Vadodara (IN)
(74) Representative: Watson, Robert James

(57) **Abstract**

The present invention provides a process for preparation of amorphous form of Lercanidipine HCl.

## Description

### Field of the Invention

The present invention provides a process for preparation of Amorphous form of Lercanidipine HCl.

### Background of the invention

Lercanidipine hydrochloride (I) is chemically known as, 1, 4-Dihydro-2, 6-dimethyl-4-(3-nitrophenyl)- 3, 5- pyridinedicarboxylic acid 2-[ (3, 3-diphenylpropyl) methylamino] -1,1-dimethylethyl methyl ester hydrochloride OR methyl 1,1, N-trimethyl- N - (3, 3-diphenylpropyl)-2-aminoethyl-1,4-dihydro-2, 6-dimethyl-4-(3-nitrophenyl)pyridine-3, 5-dicarboxylate hydrochloride OR methyl 1,1-dimethyl - 2 - [ N- (3,3-diphenylpropyl) - N - methylamino]ethyl 2,6-dimethyl-4-(3-nitrophenyl)-1,4-dihydropyridine-3,5-dicarboxylate hydrochloride having molecular formula C₃₆H₄₁N₃O₆.HCl and molecular weight 648.19. The current pharmaceutical product containing this drug has been approved for the treatment of hypertension and has been marketed since 1996 in several European countries under the trademark Zanidip(TM).

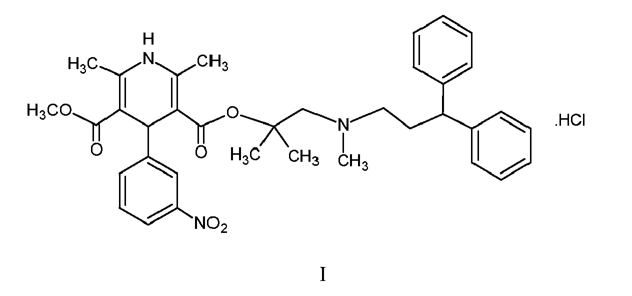

Lercanidipine HCl is useful as an anti-hypertensive. Lercanidipine HCl lowers blood pressure by blocking calcium channels of arterial smooth muscle, thus decreasing peripheral, vascular resistance. Lercanidipine HCl produces no negative cardiac in tropism and only occasional mild reflex tachycardia, which is generally of short duration.

US 4705797 discloses a process for the preparation of Lercanidipine HCl, the final step of the process being a cyclisation between 1,1,N-trimethyl-N-(3,3- diphenyl propyl)-2-aminoethyl α-acetyl-3-nitrocinnammate and methyl 3- aminocrotonate. Lercanidipine was isolated as its hydrochloride by crystallization from water containing HCl and NaCl. However, this process was expensive, time consuming, and resulted in relatively low yields of Lercanidipine hydrochloride. The product was a crude ill-defined mixture, mainly of amorphous Lercanidipine hydrochloride.

Further WO 03/014084 discloses novel crystalline forms I & II of Lercanidipine Hydrochloride and mixtures of Form I & II having predetermined and reproducible content of Form I & II & optionally other forms of Lercanidipine Hydrochloride such as amorphous. EP 1600441 discloses crystalline form I of Lercanidipine Hydrochloride and a mixture of form I with amorphous and optionally form II. However, these prior art don't describe pure amorphous form (anhydrous or hemihydrate) of Lercanidipine Hydrochloride and its preparation.

PCT Publication WO 2006/089787 discloses amorphous Lercanidipine hydrochloride having a purity of at least 95% and a method for its preparation. The method includes dissolving crystalline Lercanidipine hydrochloride in an organic solvent to provide a solution and isolating amorphous Lercanidipine hydrochloride either by (a) adding water to the solution to form a precipitate and collecting the precipitate or (b) evaporating off the organic solvent.

PCT publication WO 2007/031865 discloses novel crystalline Form V of Lercanidipine hydrochloride and use thereof for the preparation of amorphous Lercanidipine hydrochloride.

### Object of the invention

The primary object of the present invention is to provide a process for the preparation of amorphous Lercanidipine hydrochloride.

### Summary of the invention

According to one aspect of present invention, it provides a process for the preparation of amorphous form of Lercanidipine hydrochloride comprising:
i) dissolving Lercanidipine hydrochloride in acetonitrile at refluxing temperature to obtain a solution;
ii) removing acetonitrile partially or completely from the solution obtained in step i) to obtain a residue;
iii) adding suitable solvent to the residue obtained in step ii);
iv) recovering amorphous form of Lercanidipine hydrochloride.

### Brief description of the drawings

Fig. 1 depicts characteristic powder X-ray diffraction (XRD) pattern of amorphous form of Lercanidipine HCl.
Fig. 2 depicts characteristic differential scanning calorimetric (DSC) thermogram of amorphous form of Lercanidipine HCl.

### Detailed description of the invention

The term "Lercanidipine hydrochloride" refers to the hydrochloride salt of methyl 1, 1, N- trimethyl-N-(3, 3-diphenylpropyl)-2-aminoethyl 1, 4-dihydro-2, 6-dimethyl-4-(3- nitrophenyl)-pyridine-3, 5-dicarboxylate. Lercanidipine salt may be present as one or both of its enantiomeric forms.

The term "amorphous" means a solid without long-range crystalline order. Amorphous form of Lercanidipine hydrochloride in accordance with the present invention preferably contains less than about 10 percent crystalline forms of Lercanidipine hydrochloride, more preferably less than 5 percent crystalline form of Lercanidipine hydrochloride, and still more preferably is essentially free of crystalline forms of Lercanidipine hydrochloride. "Essentially free of crystalline forms of Lercanidipine hydrochloride" means that no crystalline polymorph forms of Lercanidipine hydrochloride can be detected within the limits of a powder X-ray diffractometer.

According to one general aspect of the present invention, there is provided a new, improved and industrially viable process for the preparation of Amorphous form of Lercanidipine Hydrochloride.

According to one aspect of the present invention, there is provided an efficient process for the preparation of Amorphous form of Lercanidipine Hydrochloride, which provides obvious benefits with respect to economics, generation of minimal effluents, lesser number of process steps, less reactor occupancy, higher purity of the product with convenience to operate on a commercial scale.

In one embodiment, Lercanidipine hydrochloride used as starting product may be in any solid form, i.e. amorphous, crystalline or their admixtures, preferably in crystalline form. Any polymorph or admixture of polymorphs may be used.

In step i) of the present invention, Lercanidipine HCl is treated with acetonitrile under constant stirring. This mixture is heated at the reflux temperature (80-85°C) till dissolution. The time period of the heating preferably takes place till the clear solution appears. After completing reaction the reaction mass may be allowed to cool to 40-50°C.

In step ii) of the present invention, acetonitrile is preferably partially or completely removed under vacuum at up to 50°C temperature or at atmospheric pressure from the obtained solution of step i) to obtain residue. The residue thus obtained can then degassed for 2-3 hrs at temperatures up to 50°C. The obtained residue is preferably cooled to 20-30°C.

In step iii) of the present invention, the obtained residue of step ii) is reacted with suitable solvent. Here the obtained solid can also be generated another way by adding hot cleared solution of step i) with the chilled solvent at 0-5°C. The reaction mass thus obtained by both methods is stirred at 20-30°C. The time period of the reaction can range from 2-3 hrs & the resulting solid is then preferably filtered at a temperature ranging from 0-30°C.

Here for the scope of present invention the solvent used for the step iii) is not acting as a antisolvent. The object of adding this solvent is only to obtain the reaction mass.

In step iv) of the present invention, the obtained reaction mass of step iii) is preferably washed with suitable solvent followed by drying. The solid thus obtained can be dried further under vacuum at 50-60°C for approximate 8-10 hrs to get the final product.

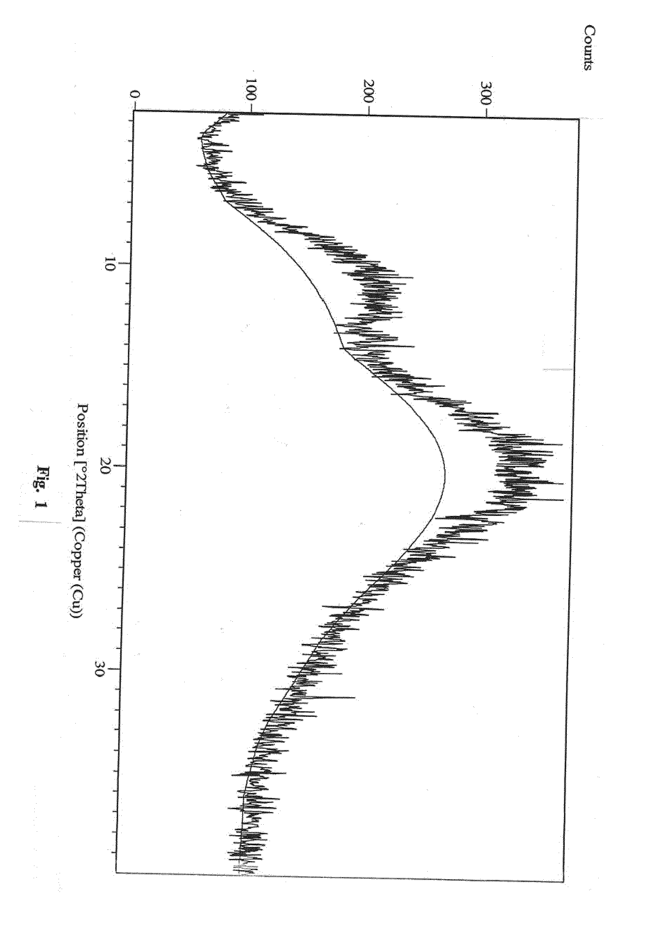

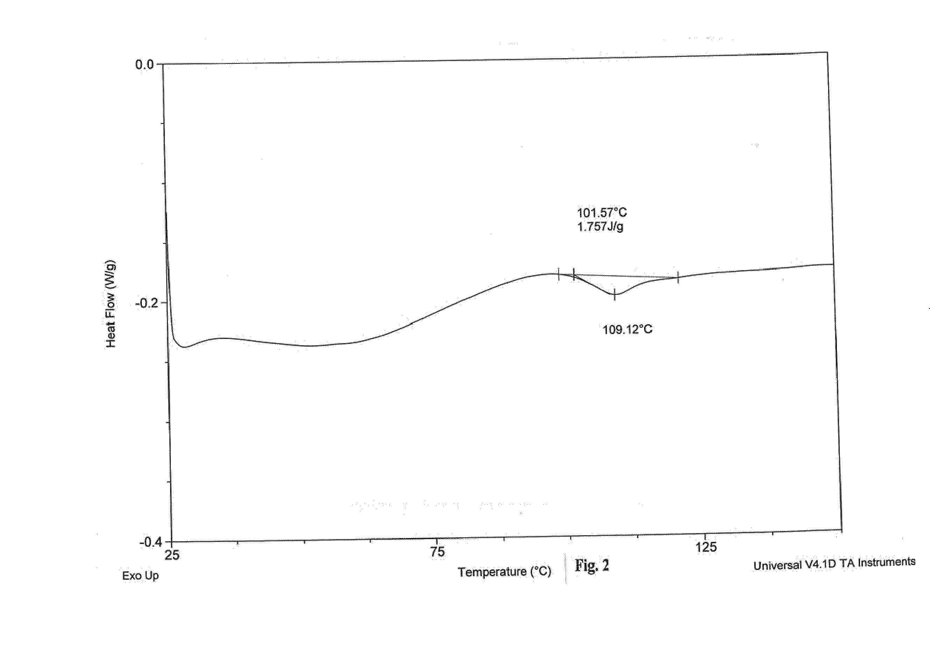

Amorphous Lercanidipine obtained as per present invention is characterized by XRD and DSC as shown in fig. 1 and 2 respectively. XRD is recorded by X-pert-PRO RDAD-1044. DSC is recorded by Universal V4.1D TA Instruments. Amorphous Lercanidipine obtained as per present invention is characterized by differential scanning calorimetry (DSC) thermogram endotherm peak occurs in the temperature range from about 108°C to about 110 °C and normally occurs at 109.1°C.

In preferred embodiment, the suitable solvent as described herein above may include, but is not limited to, non polar solvents, like cyclic or acyclic alkanes, particularly hexane, heptane, cyclohexane, pentane, cyclopentane and the like, or mixtures thereof, as well as polar solvents like water, alcohol and the like or mixtures thereof. Most preferred suitable solvents are water, n-hexane, heptane, and cyclohexane.

In the following section, embodiments are described by way of examples to illustrate the process of invention; however this does not limit the scope of the present invention. Several variants of these examples would be evident to person's skill in the art.

### Example 1

### Preparation of amorphous form of Lercanidipine HCl

20 g of Lercanidipine HCl was dissolved in 100 ml of acetonitrile under stirring at room temperature. The obtained reaction mass was heated at the reflux temperature (80-85°C) with constant stirring until the clear solution is observed. The reaction mixture was then allowed to cool to 40-50°C temperature. The acetonitrile was completely distilled out under vacuum at up to 50°C. The remaining mass is further degassed for 2-3 hrs at a temperature up to 50°C. The obtained residue is cooled to 20-30°C. Chilled (0-10°C) DM Water (200 ml) was further added to the residue & allowed to cool to 0-5°C under stirring for 2-3 hrs at same temperature. The precipitated solid was filter at 0-5°C & washed with DM Water (2 x 20 ml, 20-30°C). The product was dried under vacuum at 50-60°C for 8-10 hr. The obtained final product was amorphous Lercanidipine HCl weighing about 17.5 g.

### Example 2

### Preparation of amorphous form of Lercanidipine HCl

20 g of Lercanidipine HCl was dissolved in 100 ml of acetonitrile under stirring at room temperature. The obtainedreaction mass was heated at reflux temperature (80-85°C) with constant stirring until the clear solution is observed. The reaction mixture was then allowed to cool to 40-50°C temperature. After completion of the reaction, acetonitrile was completely distilled out under vacuum at up to 50°C. The remaining mass is further degassed for 2-3 hrs at a temperature up to 50°C followed by cooling the residue at 20-30°C. n-Hexane (200 ml) was charged in the reaction mass under stirring for 2-3 hr at 20-30°C. The precipitated solid was filter at 20-30°C & washed with n-Hexane (2 x 20 ml) at 20-30°C. The obtained solid was dried under vacuum at 50-60°C for 8-10 hr to get amorphous Lercanidipine HCl (17.5 g).

### Example 3

### Preparation of amorphous form of Lercanidipine HCl

20 g of Lercanidipine HCl was dissolved in 100 ml acetonitrile at room temperature. The obtained reaction mass was heated at reflux temperature (80-85°C) with constant stirring till the clear solution is obtained followed by cooling the reaction mass to 40-50°C. After completion of the reaction, acetonitrile was completely distilled out under vacuum up to 50°C. The remaining mass is further degassed for 2-3 hrs at a temperature up to 50°C. The residue was allowed to cool to 20-30°C. Cyclohexane (200 ml) was further added to obtain the residue at room temperature. The time period of the reaction can range from 2-3 hrs. After completion of reaction the reaction mass was filterd at RT. The obtained solid was washed with Cyclohexane (2 x 20 ml) at RT. To get the final product, the solid was dried under vacuum at 50-60°C for 8-10 hr. The obtained final product was amorphous Lercanidipine HCl weighing about 17.5 g.

### Example 4

### Preparation of amorphous form of Lercanidipine HCl

20 g of Lercanidipine HCl was dissolved in 100 ml of acetonitrile under stirring at room temperature. The obtained reaction mass was heated at the reflux temperature (80-85°C) with constant stirring until the clear solution is observed. The reaction mixture was then allowed to cool to 40-50°C temperature. The acetonitrile was completely distilled out under vacuum at up to 50°C. The remaining mass is further degassed for the 2-3 hrs at temperature up to 50°C. The obtained residue is cooled at 20-30°C. Heptane (200 ml) was further added to residue followed by stirring the reaction mass for 2-3 hr at 20-30°C. The solid was filtered at 20-30°C & washed with Heptane (2 x 20 ml) at 20-30°C temperature. The product was dried under vacuum at 50-60°C for 8-10 hr. The obtained final product was amorphous Lercanidipine HCl weighing about 17.5 g.

### Example 5

### Preparation of amorphous form of Lercanidipine HCl

20 g of Lercanidipine HCl was dissolved in 100 ml acetonitrile at room temperature. The obtained reaction mass was heated at reflux temperature (80-85°C) with constant stirring till the clear solution is obtained. DM Water (200 ml) in another cleaned & dried round bottom flask was allowed to cool at 0-5°C. The hot clear solution was added to the DM Water slowly at 0-5°C. The reaction mass was stirred for about 2-3 hrs at a temperature ranging from 0-5°C. The solids were filtered off & washed with DM Water (2 x 20 ml) at temperature ranging from 20-30°C to get the wet cake. Next the product was dried under vacuum at temperature ranging from 50-60°C for 8-10 hrs to get the final product. The purified amorphous Lercanidipine HCl weighing about 14 g.

### Example 6

### Preparation of amorphous form of Lercanidipine HCl

20 g of Lercanidipine HCl was dissolved in 100 ml acetonitrile at room temperature. The obtained reaction mass was heated at reflux temperature (80-85°C) with constant stirring till the clear solution is obtained. The Acetonitrile (60-70 ml) was distilled out atmospherically. DM Water (200 ml) in another cleaned & dried round bottom flask was allowed to cool at 0-5°C. The hot clear solution was added to the DM Water slowly at 0-5°C. The reaction mass was stirred for about 2-3 hrs at a temperature ranging from 0-5°C. The solids were filtered off & washed with DM Water (2 x 20 ml) at temperature ranging from 20-30°C to get the wet cake. Next the product was dried under vacuum at temperature ranging from 50-60°C for 8-10 hrs to get the final product. The purified amorphous Lercanidipine HCl weighing about 14 g.

## Claims

1. A process for preparation of Amorphous form of Lercanidipine HCl comprising steps of:
i) dissolving Lercanidipine hydrochloride in acetonitrile at refluxing temperature to obtain a solution;
ii) removing acetonitrile partially or completely from the solution obtained in step (i) to obtain a residue;
iii) adding suitable solvent to the residue obtained in step (ii);
iv) recovering amorphous form of Lercanidipine hydrochloride.

2. The process according to claim 1, wherein the solution obtained in step i) is allowed to cool to 40-50°C before step ii) is carried out.

3. The process according to either claim 1 or claim 2, wherein the removal of acetonitrile in step ii) is carried out by distillation.

4. The process according to claim 3, where the distillation is carried out either under vacuum at up to 50°C or at atmospheric pressure.

5. The process according to any one of claims 1 to 4, wherein the residue obtained in step ii) is degassed for 2-3 hours at a temperature up to 50°C.

6. The process according to any one of claims 1 to 5, wherein the residue obtained in step ii) is cooled to 20-30°C before step iii) is carried out.

7. The process according to any one of claims 1 to 6, wherein the suitable solvent in step iii) is selected from cyclic and acylic alkanes, water and alcohols.

8. The process according to any one of claims 1 to 6, wherein said suitable solvent in step iii) is selected from the group comprising hexane, heptane, cyclohexane, pentane, cyclopentane and water or mixtures thereof.

9. The process according to any one of claims 1 to 8, wherein in step iv), the reaction mass obtained in step iii) is washed with suitable solvent followed by drying.

10. The process according to claim 9, wherein the drying takes place under vacuum at 50-60°C for 8 to 10 hours.
